Europäisches Patentamt

European Patent Office

Office européen des brevets

(19)

(11) Numéro de publication : **0 495 717 A1**

(12)

# DEMANDE DE BREVET EUROPEEN

(21) Numéro de dépôt : 92400111.8

(22) Date de dépôt : 16.01.92

(51) Int. Cl.[5] : **C07D 487/04,** C07B 57/00, A61K 31/50, // (C07D487/04, 241:00, 209:00)

(30) Priorité : 17.01.91 FR 9100490

(43) Date de publication de la demande :
22.07.92 Bulletin 92/30

(84) Etats contractants désignés :
PT

(71) Demandeur : RHONE-POULENC RORER SA
20, avenue Raymond Aron
F-92160 Antony (FR)

(72) Inventeur : **Cotrel, Claude**
**17, avenue du Docteur Arnold Netter**
**F-75012 Paris (FR)**
Inventeur : **Roussel, Gérard**
**20 ter rue des Carrières**
**F-91450 Soisy sur Seine (FR)**

(74) Mandataire : **Pilard, Jacques et al**
**RHONE-POULENC RORER S.A. 20 Avenue**
**Raymond Aron**
**F-92165 Antony Cédex (FR)**

(54) **Dérivé de la 5H-Pyrrolo(3,4-b) pyrazine optiquement actif, sa préparation et les compositions pharmaceutiques qui le contiennent.**

(57) Isomère dextrogyre de la (chloro-5 pyridyl-2)-6 (méthyl-4 pipérazinyl-1) carbonyloxy-5 oxo-7 dihydro-6,7 5H-pyrrolo[3,4-b] pyrazine, sa préparation et les compositions pharmaceutiques qui le contiennent utilisables comme tranquillisants et hypnotiques.

EP 0 495 717 A1

EP 0 495 717 A1

Dans le brevet français FR 72 00505 publié sous le numéro 2 166 314 a été décrite en particulier la (chloro-5 pyridyl-2)-6 (méthyl-4 pipérazinyl-1) carbonyloxy-5 oxo-7 dihydro-6,7 5H-pyrrolo [3,4-b] pyrazine, connue également sous le nom de zopiclone, qui est un produit hypnotique remarquable.

Du fait de la présence d'un atome de carbone asymétrique en position -5 du cycle 5H-pyrrolo[3,4-b] pyrazine, la zopiclone doit être considérée, sous forme racémique, comme étant constituée d'un mélange rigoureusement équimoléculaire des formes lévogyre et dextrogyre.

Il a maintenant été trouvé, et c'est ce qui fait l'objet de la présente invention, que l'isomère dextrogyre de la zopiclone présente des propriétés qui ne sont pas évidentes au vu de celles de la zopiclone racémique.

La présente invention a donc pour objet l'isomère dextrogyre de la zopiclone, sa préparation et les compositions pharmaceutiques qui le contiennent. Dans un produit racémique, il est connu que souvent un des deux énantiomères est actif et que à cette activité peut être liée un accroissement de la toxicité, l'autre énantiomère étant à la fois nettement moins actif ou inactif et moins toxique. Pour de tels produits, le gain d'activité ne compense pas les inconvénients dus à une toxicité accrue.

Dans le cas de la zopiclone, il a été trouvé, de manière surprenante et inattendue, que l'isomère dextrogyre est non seulement environ 2 fois plus actif que le racémique tout en ayant une toxicité plus faible que celle du racémique et que l'isomère lévogyre est à la fois pratiquement inactif et plus toxique que le racémique.

Par exemple, chez la souris, par voie orale, la zopiclone présente une toxicité ($DL_{50}$) voisine de 850 mg/kg alors que l'isomère dextrogyre a une toxicité voisine de 1,5 g/kg et que l'isomère lévogyre présente une $DL_{50}$ comprise entre 300 et 900 mg/kg.

Chez l'animal, l'isomère dextrogyre de la zopiclone montre des propriétés hypnotique, sédative, anxiolytique, myorelaxante et anticonvulsivante.

Du point de vue de la puissance d'action dans les principaux tests mettant en évidence l'activité tranquillisante et hypnotique de la zopiclone tels que le test d'affinité vis-à-vis des sites récepteurs centraux des benzodiazépines selon la technique de J.C. Blanchard et L. Julou, J. of Neurochemistry, 40, 601 (1983) inspirée des travaux de Squires et Braestrup, Nature, 266, 732-734 (1977), le test d'activité antagoniste vis-à-vis des convulsions au pentétrazol selon la technique de Everett et Richards, J. Pharmacol., 81, 402 (1944), ou dans le test du réflexe de retournement chez la souris selon la technique de Zbinden et Randall, Advances in Pharmacology 5, 213-291 (1967), l'isomère dextrogyre est environ 2 fois plus actif alors que l'isomère lévogyre est pratiquement inactif.

Selon l'invention, l'isomère dextrogyre de la zopiclone peut être préparé à partir du racémique correspondant selon les méthodes habituelles telles que la chromatographie sur phase chirale, le dédoublement d'un sel optiquement actif ou la catalyse enzymatique stereosélective au moyen un microorganisme convenable ou par synthèse asymétrique.

Plus particulièrement, l'isomère dextrogyre de la zopiclone peut être obtenu par dédoublement de la zopiclone au moyen d'un acide optiquement actif en opérant dans un solvant organique convenable.

Comme acide optiquement actif qui convient particulièrement bien peut être cité l'acide D(+)-O,O'-dibenzoyltartrique.

Généralement, on opère dans un solvant organique choisi parmi les hydrocarbures aliphatiques halogénés tels que le dichlorométhane et les nitriles tels que l'acétonitrile pris seuls ou en mélange.

En opérant de cette manière, le sel de l'isomère dextrogyre précipite et l'isomère lévogyre est extrait des eaux-mères de cristallisation.

L'isomère dextrogyre de la zopiclone est déplacé de son sel au moyen d'une base telle que la soude.

L'isomère dextrogyre de la zopiclone est utile chez l'homme pour le traitement des états dus à un disfonctionnement du système nerveux central.

L'isomère dextrogyre de la zopiclone est, par exemple, utile comme hypnosédatif, tranquillisant, myorelaxant et anticonvulsivant.

Cependant, l'isomère dextrogyre de la zopiclone est plus particulièrement utile chez l'homme comme hypnotique.

L'isomère dextrogyre de la zopiclone agissant sur les divers paramètres du sommeil, il augmente la durée et améliore la qualité du sommeil et il diminue le nombre d'éveils nocturnes et de réveils précoces.

La présente invention concerne les compositions pharmaceutiques contenant l'isomère dextrogyre de la zopiclone ou un de ses sels pharmaceutiquement acceptables à l'état pur ou en présence d'un diluant ou d'un enrobage. Ces compositions peuvent être employées par voie orale, rectale ou parentérale.

Comme sels pharmaceutiquement acceptables peuvent être cités des sels d'acides minéraux (tels que chlorhydrates, sulfates, nitrates, phosphates) ou organiques (tels que les acétates, propionates, succinates, benzoates, fumarates, tartrates, théophyllineacétates, salicylates, phénolphtalinates, méthylène-bis-β-oxynaphtoates) ou des dérivés de substitution de ces acides.

Comme compositions solides pour administration orale peuvent être utilisés des comprimés, des pilules,

2

EP 0 495 717 A1

des poudres ou des granulés. Dans ces compositions le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale on peut utiliser des émulsions pharmaceutiquement acceptables, des solutions, des suspensions, des sirops et élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, le polyéthylèneglycol, les huiles végétales, en particulier l'huile d'olive, et les esters organiques injectables, par exemple l'oléate d'éthyle. Ces compositions peuvent contenir des adjuvants en particulier des agents mouillants, émulsifiants et dispersants. La stérilisation pot se faire de plusieurs façons, par exemple à l'aide d'un filtre bactériologique, en incorporant à la composition des agents stérilisants, par irradiation ou par chauffage. Elles peuvent être préparées sous forme de compositions stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao.

En thérapeutique humaine les doses dépendent de l'effet recherché et de la durée du traitement; elles sont généralement comprises entre 2,5 et 15 mg par jour par voie orale pour un adulte.

Les exemples suivants, donnés à titre non limitatif, illustrent la présente invention.

EXEMPLE 1

A une solution de 22,56 g d'acide D(+)-O,O′-dibenzoyltartrique sous forme de monohydrate (0,06 mole) dans 300 cm3 de dichlorométhane, on ajoute une solution de 23,28 g de zopiclone (0,06 mole) dans 300 cm3 de dichlorométhane. Le mélange réactionnel est concentré à sec sous pression réduite. Le sel brut obtenu est recristallisé dans 2000 cm3 d'acétonitrile pour donner, avec un rendement de 46 %, 21,3 g de produit cristallisé fondant à 160-165°C (avec décomposition) et dont le pouvoir rotatoire est $[\alpha]^{20}_D = 83°$ (c= 0,5 ; acétone).

Le produit obtenu est dissous dans 180 cm3 de dichlorométhane au reflux. On ajoute 200 cm3 d'acétonitrile et laisse reposer pendant 1 heure à une température de 5°C. Le produit cristallisé obtenu est à nouveau recristallisé dans les mêmes conditions. On obtient ainsi, avec un rendement de 36 %, 16,5 g de sel cristallisé fondant à 160-165°C (avec décomposition) et dont le pouvoir rotatoire est $[\alpha]^{20}_D = 102°$ (c = 0,5 ; acétone).

Le sel ainsi obtenu est dissous dans 125 cm3 d'eau en présence de 125 cm3 de dichlorométhane. Le mélange est alcalinisé à pH 11 par addition lente d'une solution aqueuse de soude 2N. Après décantation, la phase aqueuse est extraite deux fois avec du dichlorométhane. Les phases organiques réunies sont lavées à l'eau puis séchées sur sulfate de magnésium. Après filtration, évaporation du solvant et recristallisation du produit obtenu dans 80 cm3 d'acétonitrile, on obtient, avec un rendement de 23 %, 5,4 g d'isomère dextrogyre de la zopiclone fondant à 206,5°C et dont le pouvoir rotatoire est $[\alpha]^{20}_D = 135° \pm 3°$ (c = 1,0; acétone).

Les eaux-mères de cristallisation du sel de la zopiclone avec l'acide D(+)-O,O′-dibenzoyltartrique sont concentrées à sec sous pression réduite pour donner 22,05 g de sel dont le pouvoir rotatoire est $[\alpha]^{20}_D = -21°$ (c = 0,2 ; acétone).

Le sel ainsi obtenu est disous dans 125 cm3 d'eau en présence de 125 cm3 de dichlorométhane. Le mélange est alcalinisé à pH 11 par addition lente d'une solution aqueuse de soude 2N. Après décantation, la phase aqueuse est extraite deux fois avec du dichlorométhane. Les phases organiques réunies sont lavées à l'eau puis séchées sur sulfate de magnésium. Après filtration et évaporation du solvant, le solide cristallisé obtenu (8,45 g) est recristallisé successivement dans 100, 50 et 45 cm3 d'acétonitrile. On obtient ainsi, avec un rendement de 13,9 %, 3,13 g d'isomère lévogyre de la zopiclone fondant à 206,9°C et dont le pouvoir rotatoire est $[\alpha]^{20}_D = -133° \pm 3°$ (c = 1,0 ; acétone).

EXEMPLE 2

On prépare selon la technique habituelle des comprimés dosés à 3 mg de produit actif ayant la composition suivante :

3

- isomère dextrogyre de la zopiclone .............. 0,003 g

- amidon ........................................ 0,100 g

- silice précipitée ............................. 0,035 g

- stéarate de magnésium ................................. 0,005 g

## Revendications

**1** - L'isomère dextrogyre de la (chloro-5 pyridyl-2)-6 (méthyl-4 pipérazinyl-1) carbonyloxy-5 oxo-7 dihydro-6,7 5H-pyrrolo [3,4-b] pyrazine ainsi que ses sels pharmaceutiquement acceptables.

**2** - Procédé de préparation du produit selon la revendication 1 caractérisé en ce que l'on dédouble la (chloro-5 pyridyl-2)-6 (méthyl-4 pipérazinyl-1) carbonyloxy-5 oxo-7 dihydro-6,7 5H-pyrrolo[3,4-b]pyrazine racémique au moyen d'acide D(+)-O,O'-dibenzoyltartrique en opérant dans un solvant organique, isole le sel de l'isomère dextrogyre et déplace de son sel puis isole l'isomère dextrogyre de la (chloro-5 pyridyl-2)-6 (méthyl-4 pipérazinyl-1) carbonyloxy-5 oxo-7 dihydro-6,7 5H-pyrrolo[3,4-b]pyrazine que l'on transforme éventuellement en sel pharmaceutiquement acceptable.

**3** - Composition pharmaceutique caractérisée en ce qu'elle contient le produit selon la revendication 1 en association avec un ou plusieurs diluants ou adjuvants pharmaceutiquement acceptables.

**4** - Méthode pour améliorer la qualité et la durée du sommeil caractérisée en ce que l'on administre à l'homme une quantité suffisante de l'isomère dextrogyre de la zopiclone.

4

EP 0 495 717 A1

Office européen
des brevets

**RAPPORT DE RECHERCHE EUROPEENNE**

Numero de la demande

EP    92 40 0111

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-2 300 491 (RHONE-POULENC S.A) * le document en entier * | 1,3 | C07D487/04 C07B57/00 |
| D | & FR-A-2 166 314 | | A61K31/50 //(C07D487/04, |
| A | US-A-4 220 646 (C.COTREL ET AL.) * le document en entier * | 1,3 | 241:00,209:00) |
| A | 'The Merck Index,eleventh Edition' 1989 , MERCK & CO.,INC. , RATHWAY,N.J.,U.S.A Résumé 10095 * page 1605, colonne 1, alinéa 2 * | 1,3 | |

**DOMAINES TECHNIQUES RECHERCHES (Int. Cl.5)**

C07D
C07B

Le présent rapport a été établi pour toutes les revendications

| Lieu de la recherche | Date d'achèvement de la recherche | Examinateur |
|---|---|---|
| BERLIN | 20 MARS 1992 | KYRIAKAKOU G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)